(19) Europäisches Patentamt
European Patent Office
Office européen des brevets

(11) EP 2 415 456 A1

(12) EUROPEAN PATENT APPLICATION
published in accordance with Art. 153(4) EPC

(43) Date of publication:
08.02.2012 Bulletin 2012/06

(21) Application number: 10758598.6

(22) Date of filing: 28.03.2010

(51) Int Cl.:
*A61K 8/81* (2006.01) *A61K 8/34* (2006.01)
*A61Q 5/06* (2006.01) *C08F 220/26* (2006.01)

(86) International application number:
PCT/JP2010/055474

(87) International publication number:
WO 2010/113826 (07.10.2010 Gazette 2010/40)

(84) Designated Contracting States:
AT BE BG CH CY CZ DE DK EE ES FI FR GB GR HR HU IE IS IT LI LT LU LV MC MK MT NL NO PL PT RO SE SI SK SM TR

(30) Priority: 31.03.2009 JP 2009084334

(71) Applicant: Shiseido Company, Ltd.
Chuo-ku
Tokyo 104-8010 (JP)

(72) Inventors:
- TOYODA, Tomonori
Yokohama-shi
Kanagawa 224-8558 (JP)
- SHIMIZU, Hideki
Yokohama-shi
Kanagawa 224-8558 (JP)
- KURASHIMA, Takumi
Yokohama-shi
Kanagawa 224-8558 (JP)
- FUJIYAMA, Taizo
Yokohama-shi
Kanagawa 224-8558 (JP)
- MIYAZAWA, Kazuyuki
Yokohama-shi
Kanagawa 224-8558 (JP)

(74) Representative: Henkel, Breuer & Partner
Patentanwälte
Maximiliansplatz 21
80333 München (DE)

# (54) HAIR COSMETIC

(57) Disclosed is a hair cosmetic by which the hair (hair style) can be set due to the adhesiveness thereof and yet which shows an excellent hair-arranging ability (including hair-restyling ability). Specifically disclosed is a hair cosmetic comprising, as a hair-setting resin, a novel adhesive setting resin which is obtained by combining and polymerizing monomers having specific structures and shows an appropriate hardness and a high adhesiveness in a step of forming a film. In addition, this hair cosmetic preferably contains at least one component selected from a sugar, a sugar alcohol and an EO/PO derivative. Thus, a hair cosmetic having both a high hair-setting power and a good hair-arranging ability can be obtained.

EP 2 415 456 A1

## Description

### Technical Field

The present invention relates to a hair cosmetic having both the abilities to set and arrange hair (also including the ability to restyle hair). More specifically, the present invention relates to a hair cosmetic that is capable of setting hair based on fixation and restyling the hair, by virtue of a novel polymer contained therein.

### Background Art

Hair styling includes two functions: forming hairstyles and keeping the formed hairstyles. The principles on which these two functions are exerted are allegedly fixation and adhesion (Non-Patent Document 1).

Hair styling based on fixation is to set hair by forming a solid film using a film-forming agent (polymer resin) called a setting agent. For example, conventional hair gels or hair sprays are based mainly on a hair styling mechanism using a setting resin. For example, Patent Document 1 discloses a hair cosmetic mainly comprising a film-forming polymer such as polyvinylpyrrolidone, sodium polyacrylate, or a polyvinylpyrrolidone-polyvinyl acetate copolymer as a setting resin. Patent Document 2 discloses a hair cosmetic comprising a silylated urethane resin as a setting resin, and has reported that this hair cosmetic forms a film having both softness and hardness and has natural textures and a high ability to keep hairstyles.

However, the hair cosmetics obtained using the resins as described in Patent Documents 1 and 2 disadvantageously fails to restyle hair from a temporarily formed hairstyle due to a hard film formed by the setting resin and loses styling functions when the film is broken. Specifically, a problem of styling agents based on fixation brought about by such a setting resin is the poor ability to arrange hair, though they are excellent in setting hair.

On the other hand, styling based on adhesion is to allow hairs to adhere to one another by an oily ingredient. Hair liquids comprising an adhesive oily ingredient such as polyalkylene glycol as a main base, hair waxes that utilize the adhesiveness of a solid oil and have been preferred by the youth in recent years, and so on are known as such styling agents. For example, Patent Document 3 discloses a cosmetic for hair that comprises waxes and a spinnable water-soluble polymer and is excellent in restyling hair.

However, hair styling based on the adhesiveness of such an oily ingredient is characterized by being capable of restyling through fingers or a brush because the oily ingredient retains flowability and adhesiveness on the hair. A problem of this hair styling is that the ability to set hair (ability to keep hairstyles) as in hair cosmetics comprising a setting resin is not obtained, though it is excellent in the so-called ability to arrange hair.

### Prior Art Documents

#### Non-Patent Documents

Non-Patent Document 1: "Development of Advanced Cosmetics II", ed. by Masato Suzuki, published by CMC Publishing Co., Ltd., 1996, Chapter 10: Functions of Hair-Styling Agents and State-of-the-Art Technology

#### Patent Documents

Patent Document 1: JP-A-2006-213706
Patent Document 2: JP-A-2003-171244
Patent Document 3: JP-A-Hei 10-45546

### Summary of the Invention

#### Problems to be solved by the Invention

Accordingly, an object of the present invention is to provide a hair cosmetic that is capable of setting hair (hairstyle) based on fixation and is also excellent in the ability to arrange hair (also including the ability to restyle hair). The present inventors have conducted diligent studies to attain the object and consequently completed the present invention by finding that a hair cosmetic having both the abilities to set and arrange hair is obtained by formulating, as a setting resin, a novel adhesive setting resin having moderate hardness and high adhesive strength during film formation.

Means for Solving the Problems

Specifically, the present invention provides a hair cosmetic comprising an adhesive setting resin, alcohol, and water, **characterized in that** the adhesive setting resin is obtained by polymerizing:

at least one monomer represented by the following formula (A) (hereinafter, referred to as a "monomer A"):

[Formula 1]

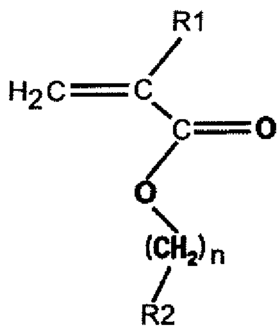

(A)

wherein R1 represents H or $CH_3$; n represents an integer of 0 to 30; $(CH_2)_n$ contains a branched chain; and R2 represents H, OH, $OCH_3$, $OCH_2CH_3$, or phenyl,
and/or

at least one monomer represented by the following formula (B) (hereinafter, referred to as a "monomer B"):

[Formula 2]

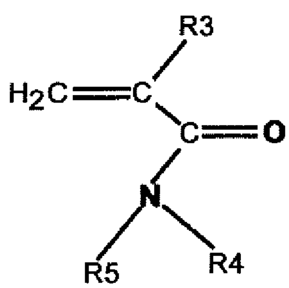

(B)

wherein R3 represents H or $CH_3$; R4 and R5, which may be the same or different, each represent H or $(CH_2)_1R'$ ; 1 represents an integer of 1 to 3; R' represents H, OH, or -NR"R'"; and R" and R'", which may be the same or different, each represent H or a C1-C3 alkyl group,
and

at least one monomer represented by the following formula (C) (hereinafter, referred to as a "monomer C"):

[Formula 3]

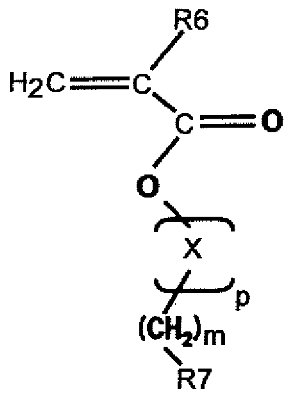

(C)

wherein R6 represents H or $CH_3$; p represents an integer of 1 to 100; m represents an integer of 0 to 30; R7 represents H, OH, $OCH_3$, $OCH_2CH_3$, or phenyl; and X represents an oxyethylene group (EO), an oxypropylene group (PO), an oxybutylene group (BO), or a glyceryl group,
and

at least one monomer represented by the following formula D (hereinafter, referred to as a "monomer D"):

[Formula 4]

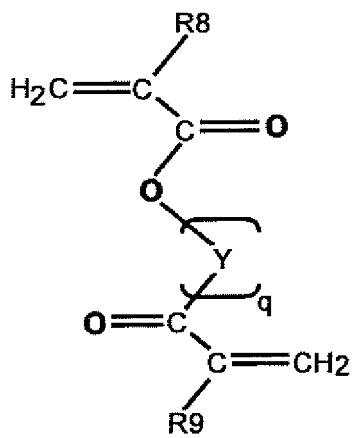

(D)

wherein R8 represents H or $CH_3$; q represents an integer of 1 to 100; and Y represents an oxyethylene group (EO), an oxypropylene group (PO), an oxybutylene group (BO), a linear or branched oxyalkylene group having 5 or more carbon atoms, or a glyceryl group, provided that q represents 1 when Y represents a linear or branched oxyalkylene group having 5 or more carbon atoms.

It is preferred that the hair cosmetic of the present invention should further comprise at least one selected from sugar, sugar alcohol, and an EO/PO derivative, in addition to the adhesive setting resin.

Effects of the Invention

A hair cosmetic of the present invention can have both the abilities to set and arrange hair, which are impossible to achieve for conventional setting resins, by formulating therein the novel adhesive setting resin described above. Furthermore, the combination of the adhesive setting resin and at least one selected from sugar, sugar alcohol, and an EO/PO derivative can further improve adhesion functions (ability to arrange hair).

Modes for Carrying out the Invention

A hair cosmetic of the present invention comprises, as an essential ingredient, an adhesive setting resin obtained by polymerizing the monomers A and/or B, and C, and D described above. Specifically, for the adhesive setting resin of the present invention, it is essential to comprise the monomers C and D. The adhesive setting resin of the present invention can comprise any one of the monomers A and B, or both. A resin (polymer) that lacks the monomer C or D produces neither favorable adhesive strength nor the ability to arrange hair (ability to restyle hair).

It is particularly preferred that the adhesive setting resin used in the present invention should have a structure represented by the following formula (I):

[Formula 5]

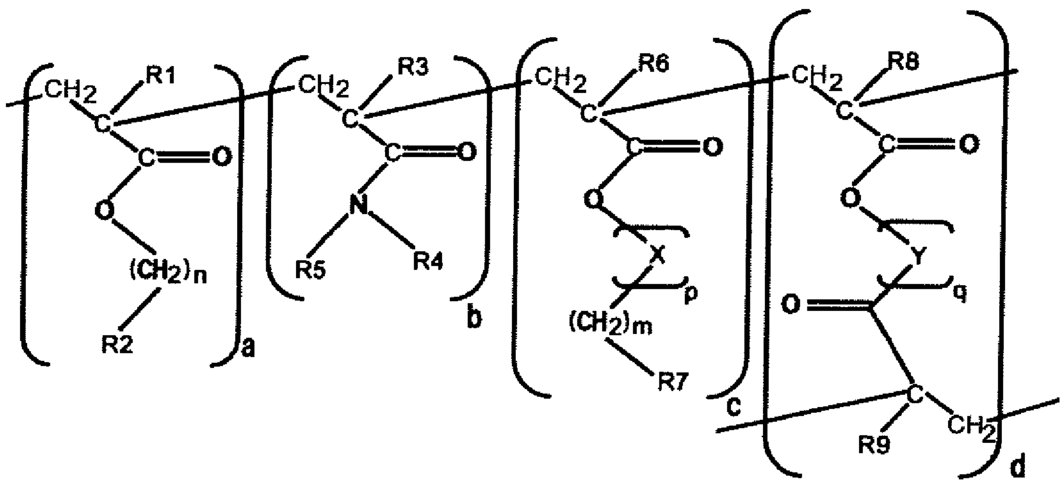

(I)

In the formula (I), R1 to R9, n, m, p, and q are as defined in the formulas A to D; a represents a number within the range of $40 < a < 400$; b represents a number within the range of $80 \leq b < 300$; c represents a number within the range of $30 < c < 300$; and d represents a number within the range of $0 < d < 10$.

The percentage by mass of each monomer in the adhesive setting resin (polymer of the formula (I)) that satisfies the conditions described above is approximately as follows: $7.5 < A < 62.5$, $20 \leq B < 45$, $7.5 < C < 60$, and $0 < D < 5$.

The adhesive setting resin of the present invention can be prepared by mixing the monomers A and/or B, and C, and D at an appropriate ratio and polymerizing the mixture through reaction using a standard method, if necessary, in an appropriate solvent. For example, the adhesive setting resin can be obtained by thermally polymerizing the mixture at approximately 80˚C for 8 hours using a polymerization initiator such as 2,2'-azobisisobutyronitrile in ethanol. The obtained polymer can be purified appropriately for use.

The amount of the adhesive setting resin formulated in the hair cosmetic of the present invention can vary depending on the product form thereof and is generally 0.1 to 30% by mass, preferably 1 to 20% by mass, more preferably 2 to 15% by mass. If the amount is less than 0.1% by mass, the ability to arrange hair may be insufficient. If the adhesive setting resin is formulated in an amount exceeding 30% by mass, the resulting hair cosmetic may make hair bristly.

The hair cosmetic of the present invention comprises alcohol and water in addition to the adhesive setting resin. One or two or more selected from alcohols generally used in cosmetics, such as ethanol, can be selected appropriately and used as the alcohol in the hair cosmetic of the present invention. The amount of the alcohol formulated is not particularly limited and can vary depending on the form of the hair cosmetic. The alcohol is usually formulated in an amount from the lower limit for use as a solvent in the adhesive setting resin to 80% by mass. Moreover, in some times, it is preferred that the amount of the alcohol formulated should be adjusted according to the amount of water formulated, in terms of controlling usability.

The content of water in the cosmetic of the present invention is usually 5 to 80% by mass, preferably 10 to 70% by mass.

It is preferred that the hair cosmetic of the present invention should further comprise at least one selected from sugar, sugar alcohol, and an EO/PO derivative. The formulation thereof can further improve the ability to arrange hair.

Examples of the sugar used in the present invention include monosaccharides and oligosaccharides. Specific examples of the monosaccharides include: triose, for example, D-glycerylaldehyde and dihydroxyacetone; tetrose, for example, D-erythrose, D-erythrulose, and D-threose; pentose, for example, L-arabinose, D-xylose, L-lyxose, D-arabinose, D-ribose, D-ribulose, D-xylulose, and L-xylulose; hexose, for example, D-glucose, D-talose, D-psicose, D-galactose, D-fructose, L-galactose, L-mannose, and D-tagatose; heptose, for example, aldoheptose and heptulose; octose, for example, octulose; deoxy sugar, for example, 2-deoxy-D-ribose, 6-deoxy-L-galactose, and 6-deoxy-L-mannose; amino sugar, for example, D-glucosamine, D-galactosamine, sialic acid, aminouronic acid, and muramic acid; and uronic acid, for example, D-glucuronic acid, D-mannuronic acid, L-guluronic acid, D-galacturonic acid, and L-iduronic acid. Further examples thereof include their derivatives (POE/POP-added, alkyl group-added, cationized, anionized, or silylated derivatives).

Examples of the oligosaccharides include sucrose, maltose, cellobiose, gentianose, umbelliferose, lactose, planteose, isolychnoses, trehalose, raffinose, lychnoses, umbilicin, stachyose verbascose. Further examples thereof include their derivatives (POE/POP-added, alkyl group-added, cationized, anionized, or silylated derivatives).

Examples of the sugar alcohol include mannitol, xylitol, erythritol, sorbitol, maltitol, and inositol. Also, their derivatives (POE/POP-added, alkyl group-added, cationized, anionized, or silylated derivatives) may be used.

The sugar and the sugar alcohol used in the present invention are not limited. Among them, sugar alcohol, particularly, maltitol or sorbitol is most preferable.

The amount of the sugar and/or the sugar alcohol formulated in the hair cosmetic of the present invention is generally 0.1 to 20% by mass, preferably 1 to 20% by mass, more preferably 3 to 10% by mass. If the amount is less than 0.1% by mass, the effect of improving the ability to arrange hair (ability to restyle hair) may be insufficient. If the sugar and/or the sugar alcohol are formulated in an amount exceeding 30% by mass, the resulting hair cosmetic may make hair sticky.

The EO/PO derivative used in the present invention is any of compounds that encompass hair-styling oils and polyalkylene glycols shown below.

The hair-styling oils mean EO/PO adducts of monohydric to tetrahydric alcohols or monovalent to trivalent carboxylic acids. Those commercially available can be used, and examples thereof can include UNILUB 50 MB-168, UNILUB MB-370, BELTAMOL P-700, BELTAMOL DG-25, TRIOL G-40, SAVON D'OR SGP-7, and SAVON D'OR GP-9 (all from NOF CORP.), and ESTEMOL 50 (Nisshin Oil Mills Co., Ltd.).

The polyalkylene glycols mean polyalkylene glycol, for example, addition polymers of ethylene oxide (EO), propylene oxide (PO), or butylene oxide (BO). Those commercially available can be used, and examples thereof can include: EO addition polymers such as PEG200, PEG300, PEG400, PEG600, PEG1000, PEG1540, PEG2000, PEG4000, PEG6000, PEG11000, and PEG20000 (NOF CORP. or TOHO Chemical Industry Co., Ltd.): and PO addition polymers such as UNIOL D-700, UNIOL D-1000, UNIOL D-1200, and UNIOL D-2000 (all from NOF CORP.).

The polyalkylene glycols used in the present invention are not limited. Among them, polyethylene glycol is most preferable.

The amount of the EO/PO derivative formulated in the hair cosmetic of the present invention is generally 0.1 to 20% by mass, preferably 1 to 20% by mass, more preferably 3 to 10% by mass. If the amount is less than 0.1% by mass, the effect of improving the ability to arrange hair (ability to restyle hair) may be insufficient. If the EO/PO derivative is formulated in an amount exceeding 30% by mass, the resulting hair cosmetic may make hair sticky.

The hair cosmetic of the present invention comprises the novel adhesive setting resin and optionally comprises at least one selected from sugar, sugar alcohol, and an EO/PO derivative. As a result, the hair cosmetic of the present invention exerts the abilities to set and arrange hair. Its form can be provided as various forms such as hair liquids, hair foams, hair mousses, hair sprays, hair mists, hair gels, and hair waxes.

The hair cosmetic of the present invention may comprise, for example, other ingredients conventionally used in hair cosmetics according to the form thereof, without impairing the effect of the present invention.

## Examples

Hereinafter, the present invention will be described in more detail with reference to specific examples. However, the present invention is not intended to be limited to Examples below. Moreover, the amount of each ingredient formulated in Examples, etc., below represents % by mass, unless otherwise specified.

(Production Examples and Comparative Production Examples)

Monomers were polymerized according to the composition shown in Table 1 below to prepare adhesive setting resins of the present invention (Production Examples 1 to 6), a monomer C-free resin of Comparative Production Example 1, and a monomer D-free resin of Comparative Production Example 2.

Specifically, a mixture of monomers mixed in the total amount of 100 parts was prepared in advance. 100 parts of ethanol were added to a 1-L five-neck flask equipped with a dropping funnel containing this mixture, a reflux condenser, a thermometer, a tube for nitrogen substitution, and a stirrer. At the point in time when the ethanol was in a reflux state (approximately 80°C) by heating under nitrogen flow, 1 part of a polymerization initiator (2,2'-azobisisobutyronitrile) was added into this ethanol, and the mixture was continuously added dropwise thereto for 2 hours. Then, the mixture was

left for 8 hours in a reflux state to allow polymerization reaction to proceed. Next, the solvent was distilled off from the solution in the five-neck flask, and ethanol was added such that the solvent content in this solution was adjusted to obtain a hair cosmetic base solution having a 50% solid content.

[Table 1]

| Classification | Chemical structure (trade name) | Manufacturer | Production Example 1 | Production Example 2 | Production Example 3 | Production Example 4 | Production Example 5 | Production Example 6 | Comparative Production Example 1 | Comparative Production Example 2 |
|---|---|---|---|---|---|---|---|---|---|---|
| A | Butyl acrylate | Idemitsu Kosan Co., Ltd. | 40 | 35 | 30 | 15 | | | | 40 |
| A | Ethyl acrylate | TOAGOSEI CO., LTD. | | | | | 30 | | 30 | |
| A | Stearyl methacrylate (BLEMMER SMA) | NOF CORP. | | | | | | | | |
| A | Hydroxyethyl acrylate (HEA) | OSAKA ORGANIC CHEMICAL INDUSTRY LTD. | | | | 15 | | | 30 | |
| A | Methoxyethyl acrylate (Arix C-1) | TOAGOSEI CO., LTD. | | | | | 15 | | | |
| B | Dimethylacrylamide (DMAA) | KOHJIN CHEMICAL CO., LTD. | | 40 | 30 | 30 | | 40 | 35 | 30 |
| B | Dimethylaminopropylacrylamide (DMAPAA) | TOAGOSEI CO., LTD. | | | | | 20 | | | |
| C | Polyoxyethylene glycol acrylate (n=10) (BLEMMER AE-400) | NOF CORP. | 55 | 20 | 15 | 15 | | | | 30 |
| C | Polyoxypropylene glycol acrylate (n=6) (BLEMMER AP-400) | NOF CORP. | | | 20 | 20 | 30 | 55 | | |
| D | Polyoxyethylene glycol diacrylate (n=23) (NK ESTER A-1000) | Shin-Nakamura Chemical Co. | 5 | 5 | 5 | 5 | | | 5 | |
| D | Polyoxyethylene glycol dimethacrylate (n=14) (NK ESTER 14G) | Shin-Nakamura Chemical Co. | | | | | | 5 | | |
| D | Glycerin dimethacrylate (BLEMMER NDMA) | NOF CORP. | | | | | 5 | | | |

(Examples and Comparative Examples)

The resins of Production Examples and Comparative Production Examples were used to prepare samples. The samples were evaluated for the ability to set hair, adhesive strength, the ability to arrange hair, the ability to restyle hair, and dryness in hair in use.
Evaluation methods and evaluation criteria for each property are shown below.

1. Ability to set hair

0.4 g of the sample was applied to black virgin hair (length: 15 cm, weight: 1 g), then spread over the hair using a comb, and styled such that the hair became straight. Five strands were prepared per sample. These strands were dried at 50˚C for 1 hour and then hung on a graduated board. A length (b) of each bent strand was measured in a thermo-hygrostat with a temperature of 30˚C and a humidity of 90% RH. The ability to set hair (ability to keep hairstyles) was determined according to a formula shown below using a length (a) of the bent strand measured in advance before application of the sample. A numeric value closer to 100% represents the higher ability to set hair and more excellent moisture resistance.

$$\text{Ability to keep hairstyles (\%)} = \{(a-b)/a\} \times 100$$

<Evaluation Criteria>

◎: The value was 90% or more.
○: The value was 70% to less than 90%.
△: The value was 50% to less than 70%.
×: The value was less than 50%.

2. Adhesive strength

0.5 g of the sample was applied to one bundle of black virgin hair (length: 20 cm, mass: 2 g). After drying at room temperature, the hair bundle was evaluated for adhesive strength on the hair in a sensory test by 10 female expert panelists.

<Criteria of evaluation scores>

5: Adhesion was considerably felt.
4: Adhesion was slightly felt.
3: Normal.
2: Adhesion was not much felt.
1: Adhesion was not felt.

<Evaluation Criteria>

◎: The total score was 40 or more.
○: The total score was 30 or more and less than 40.
△: The total score was 20 or more and less than 30.
×: The total score was less than 20.

3. Ability to arrange hair

0.5 g of the sample was applied to one bundle of black virgin hair (length: 20 cm, mass: 2 g). After drying at

room temperature, the hair bundle was evaluated for ease of arrangement in a sensory test by 10 female expert panelists.

<Criteria of evaluation scores>

5: The hair was considerably easily arranged.
4: The hair was slightly easily arranged.
3: Normal.
2: The hair was not much easily arranged.
1: The hair was difficult to arrange.

<Evaluation Criteria>

◎: The total score was 40 or more.
○: The total score was 25 or more and less than 40.
△: The total score was 20 or more and less than 25.
×: The total score was less than 20.

4. Ability to restyle hair

0.5 g of the sample was applied to one bundle of black virgin hair (length: 20 cm, mass: 2 g). After drying at room temperature, the hair bundle was evaluated for ease of restyling (ability to restyle hair) in a sensory test by 10 female expert panelists when the hair was styled immediately after the application and restyled one hour thereafter.

<Criteria of evaluation scores>

5: Considerable ability to restyle hair.
4: Slight ability to restyle hair.
3: Normal.
2: Not much ability to restyle hair.
1: No ability to restyle hair.

<Evaluation Criteria>

◎: The total score was 40 or more.
○: The total score was 25 or more and less than 40.
△: The total score was 20 or more and less than 25.
×: The total score was less than 20.

5. Absence of dryness

0.5 g of the sample was applied to one bundle of black virgin hair (length: 20 cm, mass: 2 g). After drying at room temperature, the hair bundle was evaluated for the absence of dryness during hair styling in a sensory test by 10 female expert panelists.

<Criteria of evaluation scores>

5: Hardly dry.
4: Not much dry.

3: Normal.
2: Slightly dry.
1: Dry.

<Evaluation Criteria>

◎: The total score was 40 or more.
○: The total score was 30 or more and less than 40.
△: The total score was 20 or more and less than 30.
×: The total score was less than 20.

Samples were prepared according to the composition listed in Table 2 below. The samples were prepared by adding each polymer to a mixed solution of (1) and (2) and stirring the mixture. Subsequently, the properties of each sample were evaluated according to the criteria described above. The results are also shown in Table 2.

[Table 2]

| | Comparative Example 1 | Comparative Example 2 | Comparative Example 3 | Example 1 | Example 2 | Example 3 | Example 4 | Example 5 | Example 6 |
|---|---|---|---|---|---|---|---|---|---|
| (1) Water | 65 | 60 | 60 | 60 | 60 | 60 | 60 | 60 | 60 |
| (2) Ethanol | 35 | 35 | 35 | 35 | 35 | 35 | 35 | 35 | 35 |
| (3) Comparative Production Example 1 | | 5 | | | | | | | |
| (4) Comparative Production Example 2 | | | 5 | | | | | | |
| (5) Production Example 1 | | | | 5 | | | | | |
| (6) Production Example 2 | | | | | 5 | | | | |
| (7) Production Example 3 | | | | | | 5 | | | |
| (8) Production Example 4 | | | | | | | 5 | | |
| (9) Production Example 5 | | | | | | | | 5 | |
| (10) Production Example 6 | | | | | | | | | 5 |
| Total | 100 | 100 | 100 | 100 | 100 | 100 | 100 | 100 | 100 |

(continued)

| | Comparative Example 1 | Comparative Example 2 | Comparative Example 3 | Example 1 | Example 2 | Example 3 | Example 4 | Example 5 | Example 6 |
|---|---|---|---|---|---|---|---|---|---|
| | | | | | | | | | |
| Ability to set hair | × | ○ | ○ | ○ | ○ | ○ | ○ | ○ | Δ |
| Adhesive strength | × | × | × | ○ | ○ | ○ | ○ | ○ | ○ |
| Ability to arrange hair | × | × | × | ○ | ○ | ○ | ○ | ○ | ○ |
| Ability to restyle hair | × | × | × | ○ | ○ | ◎ | ◎ | ○ | ○ |
| Dryness | ○ | ○ | ○ | ○ | ○ | ○ | ○ | ○ | ○ |

As is evident from the results shown in Table 2, the setting resin-free sample (Comparative Example 1) and the samples (Comparative Examples 2 and 3) comprising the monomer C- or D-free resin (Comparative Production Examples 1 and 2) were much inferior in adhesive strength, the ability to arrange hair, and the ability to restyle hair to the samples of Examples 1 to 6 comprising the adhesive setting resin of the present invention.

Samples were prepared according to the composition listed in Table 3 below, and the properties of each sample were evaluated according to the criteria described above. The results are also shown in Table 3.

[Table 3]

| | Comparative Example 1 | Example 3 | Example 7 | Example 8 | Example 9 | Example 10 |
|---|---|---|---|---|---|---|
| (1) Water | 65 | 60 | 64.9 | 64 | 55 | 45 |
| (2) Ethanol | 35 | 35 | 35 | 35 | 35 | 35 |
| (3) Production Example 3 | 0 | 5 | 0.1 | 1 | 10 | 20 |
| Total | 100 | 100 | 100 | 100 | 100 | 100 |
| | | | | | | |
| Ability to set hair | × | ○ | Δ | ○ | ○ | ○ |
| Adhesive strength | × | ○ | Δ | ○ | ◎ | ◎ |
| Ability to arrange hair | × | ○ | Δ | ○ | ◎ | ○ |
| Ability to restyle hair | × | ◎ | ○ | ○ | ◎ | ◎ |
| Dryness | ○ | ○ | ○ | ○ | ○ | Δ |

The results shown in Table 3 demonstrated that the ability to arrange hair and the ability to restyle hair were exerted by formulating 0.1% by mass or more of the adhesive setting resin (Production Example 3) of the present invention (Examples 3 and 7 to 10).

Samples were prepared according to the composition (wherein an EO/PO derivative was formulated in addition to an adhesive setting resin) listed in Table 4 below. The preparation method involved adding (4) to a mixed solution of (1) and (2), stirring the mixture, then adding (3) thereto, and further stirring the mixture. The properties of each sample were evaluated according to the criteria described above. The results are also shown in Table 4.

[Table 4]

| | Example 3 | Example 11 | Example 12 | Example 13 | Example 14 |
|---|---|---|---|---|---|
| (1) Water | 60 | 59 | 55 | 50 | 40 |
| (2) Ethanol | 35 | 35 | 35 | 35 | 35 |
| (3) Production Example 3 | 5 | 5 | 5 | 5 | 5 |
| (4) PPG-70 polyglyceryl-10 | | 1 | 5 | 10 | 20 |
| Total | 100 | 100 | 100 | 100 | 100 |
| | | | | | |
| Ability to set hair | ○ | ○ | ○ | Δ | Δ |
| Adhesive strength | ○ | ○ | ◎ | ◎ | ◎ |
| Ability to arrange hair | ○ | ○ | ○ | ○ | ○ |
| Ability to restyle hair | ◎ | ◎ | ◎ | ○ | Δ |
| Dryness | ○ | ○ | ◎ | ◎ | ◎ |

The samples of Examples 12 to 14 comprising the EO/PO derivative exhibited excellent properties particularly

in terms of adhesive strength, the ability to restyle hair, and the absence of dryness, compared with the EO/PO derivative-free sample of Example 3.

Samples were prepared according to the composition (wherein sugar alcohol was formulated in addition to an adhesive setting resin) listed in Table 5 below. The preparation method involved adding (4) to (1), stirring the mixture, then adding (2) thereto, stirring the mixture, finally adding (3) thereto, and stirring the mixture. The properties of each sample were evaluated according to the criteria described above. The results are also shown in Table 5.

[Table 5]

| | Example 3 | Example 15 | Example 16 | Example 17 | Example 18 |
|---|---|---|---|---|---|
| (1) Water | 60 | 59 | 55 | 50 | 40 |
| (2) Ethanol | 35 | 35 | 35 | 35 | 35 |
| (3) Production Example 3 | 5 | 5 | 5 | 5 | 5 |
| (4) maltitol | | 1 | 5 | 10 | 20 |
| Total | 100 | 100 | 100 | 100 | 100 |
| | | | | | |
| Ability to set hair | ○ | ○ | ○ | ○ | ○ |
| Adhesive strength | ○ | ○ | ◎ | ◎ | ◎ |
| Ability to arrange hair | ○ | ○ | ◎ | ◎ | ◎ |
| Ability to restyle hair | ◎ | ◎ | ◎ | ◎ | ◎ |
| Dryness | ○ | ○ | ○ | ○ | Δ |

The samples of Examples 16 to 18 comprising the sugar alcohol exhibited excellent properties particularly in terms of adhesive strength, the ability to arrange hair, and the ability to restyle hair, compared with the sugar alcohol-free sample of Example 3.

Samples were prepared according to the composition (wherein two EO/PO derivatives were formulated in addition to an adhesive setting resin) listed in Table 6 below. The preparation method involved adding (4) and (5) to a mixed solution of (1) and (2), stirring the mixture, then adding (3) thereto, and further stirring the mixture. The properties of each sample were evaluated according to the criteria described above. The results are also shown in Table 6.

[Table 6]

| | Example 3 | Example 19 | Example 20 | Example 21 | Example 22 |
|---|---|---|---|---|---|
| (1) Water | 60 | 59 | 50 | 40 | 30 |
| (2) Ethanol | 35 | 35 | 35 | 35 | 35 |
| (3) Production Example 3 | 5 | 5 | 5 | 5 | 5 |
| (4) PEG-6 | | 0.5 | 5 | 10 | 15 |
| (5) PEG-32 | | 0.5 | 5 | 10 | 15 |
| Total | 100 | 100 | 100 | 100 | 100 |
| | | | | | |
| Ability to set hair | ○ | ○ | ○ | ○ | ○ |
| Adhesive strength | ○ | ○ | ◎ | ◎ | ◎ |
| Ability to arrange hair | ○ | ○ | ◎ | ◎ | ◎ |
| Ability to restyle hair | ◎ | ◎ | ◎ | ◎ | ◎ |
| Dryness | ○ | ○ | ○ | ○ | Δ |

The samples of Examples 20 to 22 comprising the EO/PO derivatives exhibited excellent properties particularly in terms of adhesive strength, the ability to arrange hair, and the ability to restyle hair, compared with the EO/PO derivative-free sample of Example 3.

The other Examples are shown below.

(Example 23)

| Liquid styling agent | |
|---|---|
| (1) ion-exchanged water | balance |
| (2) PEG-6 | 5 |
| (3) PEG-8 | 5 |
| (4) PEG-32 | 5 |
| (5) sorbitol | 3 |
| (6) ethanol | 35 |
| (7) fragrance | q.s. |
| (8) polymer obtained in Production Example 3 | 5 |
| (9) (alkyl acrylate/diacetone acrylamide) copolymer | 2.5 |
| (10) citric acid | q.s. |

<Production Process>

Water-soluble ingredients (2) to (5) were added to water (1) and dissolved by stirring to prepare aqueous parts. Next, (7) was added to (6), and the mixture was stirred for solubilization. Then, (8) and (9) were added thereto, and the mixture was stirred to prepare alcohol parts. The aqueous parts and the alcohol parts were mixed, and (10) was added to the mixture to obtain a liquid styling agent.

(Example 24)

| Hair liquid | |
|---|---|
| (1) ethanol | 55 |
| (2) propylene glycol | 5 |
| (3) polyoxyethylene polyoxypropylene pentaerythritol ether (5EO) | 25 |
| (4) 2-ethylhexyl p-methoxycinnamate | 0.5 |
| (5) polymer obtained in Production Example 3 | 5 |
| (6) dye | q.s. |
| (7) ion-exchanged water | balance |
| (8) fragrance | q.s. |

<Production Process>

(2) to (5) were added to (1) and dissolved by well stirring. Next, (8) was added thereto, and the mixture was solubilized to prepare alcohol parts. On the other hand, (6) was dissolved in (7) by stirring, and this solution was used as aqueous parts and mixed with the alcohol parts to obtain a hair liquid.

(Example 25)

| Hair wax | |
|---|---|
| (1) candelilla wax | 2 |

(continued)

| Hair wax | |
|---|---|
| (2) microcrystalline wax | 12 |
| (3) liquid paraffin | 3.5 |
| (4) hydrogenated polyisobutene | 3.5 |
| (5) pentaerythrityl tetraethylhexanoate | 3 |
| (6) PEG-60 glyceryl isostearate | 1 |
| (7) glyceryl stearate | 1 |
| (8) behenyl alcohol | 3.3 |
| (9) stearyl alcohol | 0.9 |
| (10) tocopherol | 0.5 |
| (11) fragrance | q.s. |
| (12) ion-exchanged water | balance |
| (13) PG | 8 |
| (14) sodium stearoyl methyl taurate | 1.2 |
| (15) silicic acid anhydride | 2.5 |
| (16) TEA | 0.4 |
| (17) polymer obtained in Production Example 3 | 3 |
| (18) ethanol | 5 |

<Production Process>

(1) to (11) were dissolved by stirring at 80 to 90˚C, and this solution was used as oil-phase parts. (12) to (14) were dissolved by stirring at 70˚C to 80˚C, and this solution was used as aqueous-phase parts. The oil-phase parts were added to the aqueous-phase parts, and the mixture was emulsified using a homo mixer. Then, (15) was added to the emulsion. After neutralization by the addition of (16), (17) and (18) were added thereto, and the mixture was degassed and cooled to obtain a hair wax.

(Example 26)

| Hair wax | |
|---|---|
| (1) methylpolysiloxane | 2.0 |
| (2) microcrystalline wax | 12.0 |
| (3) liquid paraffin | 3.5 |
| (4) hydrogenated polyisobutene | 3.5 |
| (5) pentaerythrityl tetra-2-ethylhexanoate | 3.0 |
| (6) PEG-60 glyceryl isostearate | 1.0 |
| (7) glyceryl stearate | 1.0 |
| (8) deodorized cetanol (derived from plant oil) | 3.3 |
| (9) stearyl alcohol | 0.9 |
| (10) tocopherol | 0.5 |
| (11) fragrance | 0.1 |
| (12) ion-exchanged water | balance |
| (13) propylene glycol | 8.0 |
| (14) stearyl trimethyl ammonium chloride | 1.2 |
| (15) kaolin | 2.5 |
| (16) triethanolamine | 0.4 |
| (17) polyvinylpyrrolidone-vinyl acetate copolymer | 1.8 |
| (18) polymer obtained in Production Example 3 | 2.0 |
| (19) ethanol | 5.0 |

<Production Process>

(1) to (11) were dissolved by stirring at 85˚C (oil-phase parts). On the other hand, (12) to (14) were dissolved by stirring at 75˚C (aqueous-phase parts). The oil-phase parts were added to the aqueous-phase parts, and the mixture was emulsified. Then, (15) was added to the emulsion. Subsequently, after neutralization by the addition of (16), (17), (18), and (19) were added thereto, and the mixture was degassed and cooled.

(Example 27)

| Hair wax | |
|---|---|
| (1) kaolin | 1.0 |
| (2) volatile isoparaffin | 5.0 |
| (3) cetyl octanoate | 5.0 |
| (4) phenylmethylpolysiloxane | 0.5 |
| (5) candelilla wax | 3.0 |
| (6) paraffin wax | 8.0 |
| (7) propylene glycol | 5.0 |
| (8) glyceryl stearate | 1.0 |
| (9) polyoxyethylene glycerin monostearate(5EO) | 1.0 |
| (10) isostearic acid | 1.0 |
| (11) carboxyvinyl polymer | 0.4 |
| (12) potassium hydroxide (adjusted to pH 7.5) | q.s. |
| (13) ion-exchanged water | balance |
| (14) polymer obtained in Production Example 3 | 5.0 |
| (15) sorbitol | 3.0 |
| (16) EDTA-2Na·$2H_2O$ | 0.05 |
| (17) phenoxyethanol | 0.5 |
| (18) fragrance | q.s. |
| (19) highly polymerized polyethylene glycol | 0.1 |
| (20) ethanol | 5.0 |

<Production Process>

(16), (7), and (15) were added to (13) and dissolved. Then, (11) was added to the solution and uniformly dispersed by stirring. (1) was added thereto and uniformly dispersed using Disper. The mixture was heated to 85˚C, and a mixture of (2) to (9) dissolved by stirring at 85˚C in the same way as above was then added thereto and uniformly stirred. Then, (12) was added thereto, and the mixture was emulsified using a homo mixer. (14), (15), and (17) to (20) were sequentially added thereto, and the mixture was cooled to 25˚C to obtain a hair wax.

(Example 28)

| Hair wax | |
|---|---|
| (1) talc | 1.0 |
| (2) liquid paraffin | 5.0 |
| (3) pentaerythrityl tetraethylhexanoate | 5.0 |
| (4) polyether-modified methylpolysiloxane | 0.5 |
| (5) carnauba wax | 3.0 |
| (5) polyethylene wax | 8.0 |
| (7) dipropylene glycol | 5.0 |
| (8) polyoxyethylene hydrogenated castor oil (60EO) | 2.0 |

(continued)

| Hair wax | |
|---|---|
| (9) isostearic acid | 1.0 |
| (10) stearyl alcohol | 1.0 |
| (11) (PEG-240/decyltetradeceth-20/HDI) copolymer | 2.0 |
| (12) triethanolamine (adjusted to pH 7.5) | q.s. |
| (13) ion-exchanged water | balance |
| (14) polymer obtained in Production Example 3 | 10.0 |
| (15) (alkyl acrylate/diacetone acrylamide) copolymer | 1 |
| (16) 2-alkyl-N-carboxymethyl-N-hydroxyethyl imidazolinium betaine | 5.0 |
| (17) phenoxyethanol | 0.5 |
| (18) fragrance | q.s. |
| (19) highly polymerized sodium polyacrylate | 0.1 |
| (20) ethanol | 5.0 |

<Production Process>

A hair wax was obtained in the same way as in Example 26.

(Example 29)

| Hair-styling gel | |
|---|---|
| (1) carboxyvinyl polymer | 0.7 |
| (2) water dispersion of urethane resin of Production Example 2 (resin content: 40% by mass) | 5.0 |
| (3) glycerin | 2.5 |
| (4) 1,3-butylene glycol | 2.5 |
| (5) polyoxyethylene octyldodecyl ether (20EO) | 0.5 |
| (6) polyether-modified dimethylpolysiloxane | 1.0 |
| (7) sodium hydroxide (adjusted to pH 7.5) | q.s. |
| (8) ethanol | 20.0 |
| (9) polyoxyethylene octyldodecyl ether | 0.1 |
| (10) fragrance | 0.1 |
| (11) trisodium edetate | 0.03 |
| (12) ion-exchanged water | balance |
| (13) polymer obtained in Production Example 3 | 5.0 |

<Production Process>

(6) was added to (3), (4), (5), and a portion of (12), and the mixture was emulsified using a homo mixer. Subsequently, a portion of the remaining (12) was added to the emulsion to prepare emulsified parts. On the other hand, (1), (2), (7), (8), (9), (10), (11), and (13) were uniformly dissolved in the remaining portion of (12). To this solution, the emulsified parts were added to obtain a hair-styling gel emulsion.

(Example 30)

| Hair-styling gel | |
|---|---|
| (1) (PEG-240/decyltetradeceth-20/HDI) copolymer | 2.0 |
| (2) polymer obtained in Production Example 3 | 1.0 |
| (3) diglycerin | 2.5 |

(continued)

| | |
|---|---|
| Hair-styling gel | |
| (4) polyethylene glycol 1000 | 2.5 |
| (5) polyoxyethylene hydrogenated castor oil (40EO) | 0.5 |
| (6) dimethylpolysiloxane modified with hydroxy at both ends (1,000,000 mPa·s) | 1.0 |
| (7) sodium hydroxide (adjusted to pH 7.5) | q.s. |
| (8) ethanol | 20.0 |
| (9) polyoxyethylene octyldodecyl ether | 0.1 |
| (10) fragrance | 0.1 |
| (11) trisodium edetate | 0.03 |
| (12) ion-exchanged water | balance |
| (13) (alkyl acrylate/diacetone acrylamide) copolymer | 1.0 |

<Production Process>

A hair-styling gel was produced according to Example 29.

(Example 31)

| | |
|---|---|
| Styling mousse | |
| (1) dimethylpolysiloxane (20 mpa·s) | 5.0 |
| (2) isoparaffin | 5.0 |
| (3) high-molecular-weight polysiloxane | 2.0 |
| (4) amino-modified high-molecular-weight silicone | 0.5 |
| (5) 1,3-butylene glycol | 3.0 |
| (6) polyoxyethylene hydrogenated castor oil (40EO) | 2.0 |
| (7) polymer obtained in Production Example 3 | 10.0 |
| (8) polyoxyethylene/polyoxypropylene decyl ether (12EO·2PO) | 1.0 |
| (9) behenyl trimethyl ammonium chloride | 0.1 |
| (10) phenoxyethanol | 0.1 |
| (11) ethanol | 8.0 |
| (12) ion-exchanged water | balance |
| (13) fragrance | q.s. |
| (14) (octylacrylamide/hydroxypropyl acrylate/butylaminoethyl methacrylate) copolymer | 1.0 |

<Production Process>

(3) and (4) were dissolved in (1) and (2) by stirring, and this solution was added to (5), (6), and a portion of (12). The mixture was emulsified using a homo mixer (emulsified parts). On the other hand, (7) was added to the remaining portion of (12) (aqueous-phase parts). (8), (9), (10), (13), and (14) were added to (11) and dissolved by stirring. This solution was added to the aqueous-phase parts, to which the emulsified parts were further added and uniformly mixed to prepare a stock solution. 90 parts of this stock solution were charged into a can for aerosol. The can was valved, and 10 parts of liquefied petroleum gas (LPG) were charged thereto to obtain a styling mousse.

(Example 32)

| | |
|---|---|
| Styling mousse | |
| (1) dimethylpolysiloxane (6 mPa·s) | 5.0 |
| (2) isoparaffin | 5.0 |

(continued)

| | |
|---|---|
| Styling mousse | |
| (3) (PEG/amodimethicone) copolymer | 1.0 |
| (4) 1,3-butylene glycol | 3.0 |
| (5) polyoxyethylene hydrogenated castor oil (40EO) | 2.0 |
| (6) polymer obtained in Production Example 3 | 1.0 |
| (7) lauric acid diethanolamide | 0.2 |
| (8) stearoxy hydroxypropylamine | 0.1 |
| (9) phenoxyethanol | 0.1 |
| (10) ethanol | 8.0 |
| (11) ion-exchanged water | balance |
| (12) fragrance | q.s. |
| (13) (octylacrylamide/hydroxypropyl acrylate/butylaminoethyl methacrylate) copolymer | 0.5 |

<Production Process>

A styling mousse was produced according to Example 31.

## Claims

1. A hair cosmetic comprising an adhesive setting resin, alcohol, and water, **characterized in that** the adhesive setting resin is obtained by polymerizing:

at least one monomer represented by the following formula (A) :

[Formula 1]

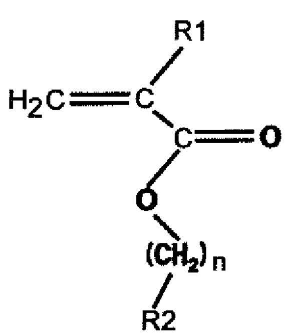

(A)

wherein R1 represents H or $CH_3$; n represents an integer of 0 to 30; $(CH_2)_n$ contains a branched chain; and R2 represents H, OH, $OCH_3$, $OCH_2CH_3$, or phenyl,
and/or
at least one monomer represented by the following formula (B) :

[Formula 2]

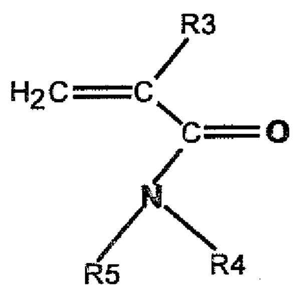

(B)

wherein R3 represents H or $CH_3$; R4 and R5, which may be the same or different, each represent H or $(CH_2)_1R'$; 1 represents an integer of 1 to 3; R' represents H, OH, or -NR"R'"; and R" and R'", which may be the same or different, each represent H or a C1-C3 alkyl group,
and
at least one monomer represented by the following formula (C) :

[Formula 3]

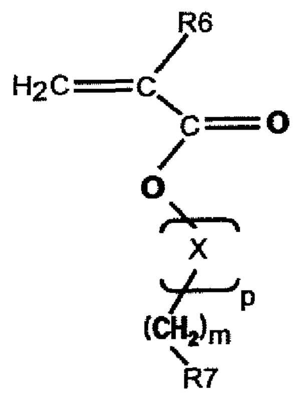

(C)

wherein R6 represents H or $CH_3$; p represents an integer of 1 to 100; m represents an integer of 0 to 30; R7 represents H, OH, $OCH_3$, $OCH_2CH_3$, or phenyl; and X represents an oxyethylene group (EO), an oxypropylene group (PO), an oxybutylene group (BO), or a glyceryl group,
and
at least one monomer represented by the following formula D:

[Formula 4]

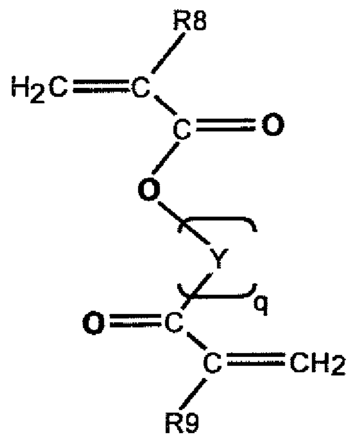

(D)

wherein R8 represents H or $CH_3$; q represents an integer of 1 to 100; and Y represents an oxyethylene group (EO), an oxypropylene group (PO), an oxybutylene group (BO), a linear or branched oxyalkylene group having 5 or more carbon atoms, or a glyceryl group, provided that q represents 1 when Y represents a linear or branched oxyalkylene group having 5 or more carbon atoms.

**2.** The hair cosmetic according to claim 1, wherein the adhesive setting resin has a structure represented by the following formula (I):

[Formula 5]

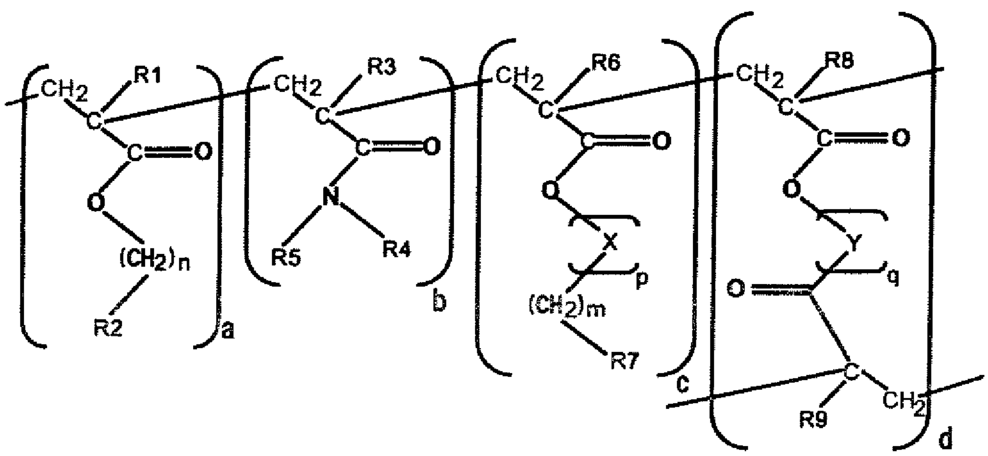

(I)

wherein R1 to R9, n, m, p, and q are as defined in the formulas A to D;
a represents a number within the range of $40 < a < 400$;
b represents a number within the range of $80 \leq b < 300$;
c represents a number within the range of $30 < c < 300$;
and
d represents a number within the range of $0 < d < 10$.

**3.** The hair cosmetic according to claim 1 or 2, wherein the hair cosmetic further comprises at least one selected from sugar, sugar alcohol, and an EO/PO derivative.

**4.** The hair cosmetic according to claim 3, wherein the sugar alcohol is selected from maltitol and sorbitol.

**5.** The hair cosmetic according to claim 3, wherein the EO/PO derivative is polyethylene glycol.

## INTERNATIONAL SEARCH REPORT

International application No.
PCT/JP2010/055474

A. CLASSIFICATION OF SUBJECT MATTER
*A61K8/81*(2006.01)i, *A61K8/34*(2006.01)i, *A61Q5/06*(2006.01)i, *C08F220/26*(2006.01)i

According to International Patent Classification (IPC) or to both national classification and IPC

B. FIELDS SEARCHED

Minimum documentation searched (classification system followed by classification symbols)
A61K8/81, A61K8/34, A61Q5/06, C08F220/26

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 6-316510 A (Osaka Organic Chemical Ind.Co., Ltd.), 15 November 1994 (15.11.1994), claims; paragraphs [0042], [0069], [0081], [0086], [0127], [0128], [0131] (Family: none) | 1-5 |
| X | JP 2002-322219 A (Lion Corp.), 08 November 2002 (08.11.2002), claims; paragraphs [0047], [0062] to [0065], [0079], [0081] (Family: none) | 1-5 |
| A | JP 2005-213448 A (Pola Chemical Industries Inc.), 11 August 2005 (11.08.2005), (Family: none) | 1-5 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 April, 2010 (08.04.10) | 20 April, 2010 (20.04.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 2006213706 A
- JP 2003171244 A
- JP HEI1045546 A

### Non-patent literature cited in the description

- Functions of Hair-Styling Agents and State-of-the-Art Technology. Development of Advanced Cosmetics II. CMC Publishing Co., Ltd, 1996